# EUROPEAN PATENT SPECIFICATION

(11) **EP 1 046 705 B1**
(45) Date of publication and mention of the grant of the patent: **26.06.2002**
(21) Application number: 00108547.1
(22) Date of filing: 19.04.2000
(51) Int. Cl.: C12M 1/00, B01L 7/00

(54) **Interior housing of humidistat**
Gehäuse für Feuchtigkeitregler
Enceinte pour régulateur d'humidité

(30) Priority: 23.04.1999 JP 11687899
(43) Date of publication of application: 25.10.2000
(73) Proprietor: Sanyo Electric Co., Ltd., Moriguchi-shi, Osaka 570-0083 (JP)
(72) Inventor: Saga, Tadahisa, Ora-gun, Gunma 370-0533 (JP); Busujima, Hiroki, Ota-shi, Gunma 373-0021 (JP); Tamaoki, Yuichi, Ora-gun, Gunma 370-0518 (JP)
(74) Representative: Glawe, Delfs, Moll & Partner

(56) References cited:
- EP-A- 0 238 313
- US-A- 2 124 250
- US-A- 4 689 303
- PATENT ABSTRACTS OF JAPAN vol. 006, no. 139 (C-116), 28 July 1982 (1982-07-28) & JP 57 063666 A (NISSHIN STEEL CO LTD), 17 April 1982 (1982-04-17)

## Description

### BACKGROUND OF THE INVENTION

### Field of the Invention

The present invention relates to an interior housing of a chamber such as a CO₂ incubator, an incubator, a thermostat/humidistat, an environmental tester, a food preserver, a food high-humidity chamber and others which can produce an environment where cells or microbes can easily breed.

### Description of the Prior Art

An incubator or food high-humidity chamber is strictly required to have cleanliness of the interior thereof in order to keep contents clean.

Further, the interior environment is under extreme conditions where bacteria and rust are apt to be generated. In particular, since the CO₂ incubator generates the temperature/humidity/CO₂ gas environment in the interior thereof, water droplets adhere to the interior housing. The water droplets can easily produce rust in the interior housing.

The bacteria in the air adheres to and breeds at the portion having the water drops adhered thereto in the chamber, which can be a cause of contamination of cultured beings. In addition, the damp air or the water droplet in the chamber is acidified by the CO₂ gas supplied to the inside of the chamber, enhancing the causticity.

For the meantime, there is known a fact that copper has an antibacterial action. Although it is known that any material other than copper, e.g., silver also has the antibacterial action and the corrosion resistance, it is very expensive and it is not commercially practical. Paying attention to the antibacterial action of the copper, a new steel sheet which is based on stainless steel and includes a copper component (so-called antibacterial stainless) has been produced.

This is presented in Japanese Patent Application Laid-Open Nos. 60302/1996, 53738/1993, 104953/1996, 229107/1996 and others.

Further, an incubator using such a steel sheet is introduced in Japanese Patent Application Laid-Open No. 234351/1998.

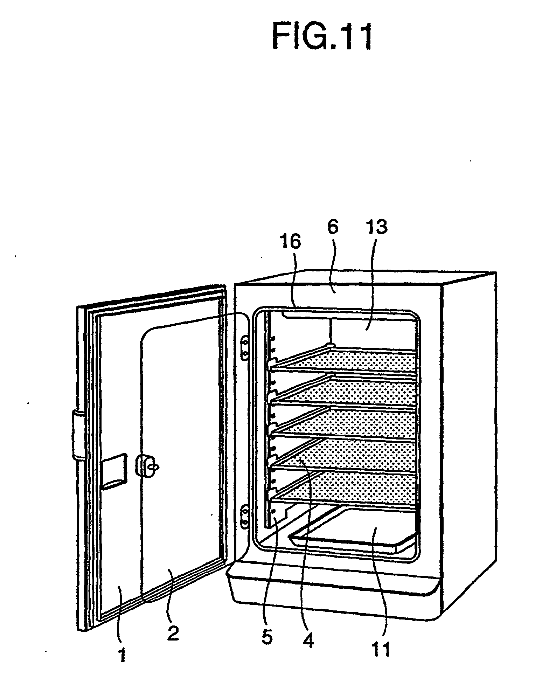

The exterior view of such a CO₂ incubator is shown in Fig. 11.

Reference numeral 6 denotes an external housing; 1, an outer door; 2, an inner door; 16, a gasket; 13, an interior housing.

Reference numeral 11 designates a reservoir in which humidification water is filled for keeping the high humidity in the chamber.

When the inner door 2 is closed so as to be appressed against the gasket 16, the inside of the chamber which is a sealed space is formed by the inner door 2 and the interior housing 13.

In addition, when the outer door 1 is closed so as to be appressed against the external housing 6, a temperature in the chamber can be safely controlled. Columns 5 and shelves 4 for supporting the cultured beings are attached to the interior of the chamber if necessary.

The CO₂ incubator should generally used while constantly keeping its interior clean, and the regular cleaning or sterilization is usually carried out. This regular cleaning or sterilization is neglected, mold or bacteria propagates itself.

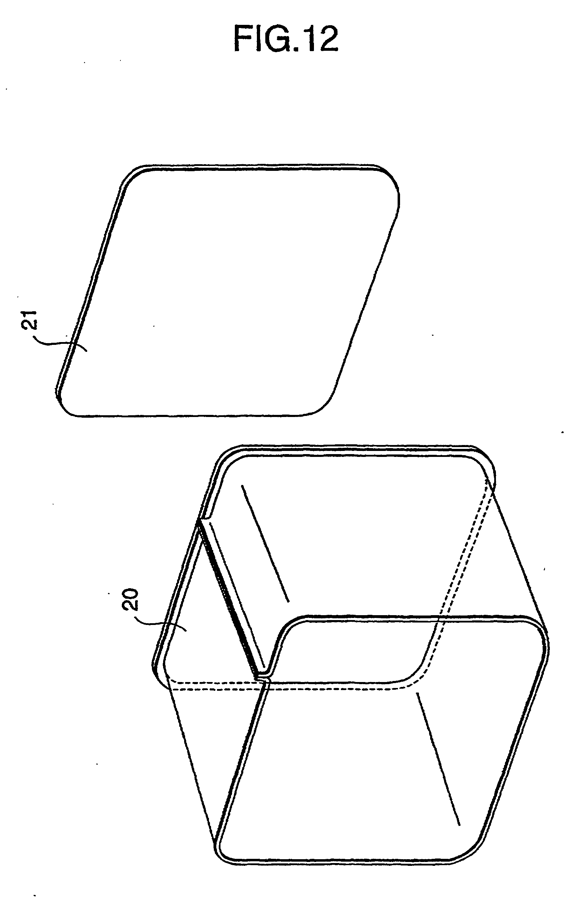

Welding of the prior art interior housing 13 will now be described with reference to Figs. 12 to 14.

The interior housing structure is obtained by welding and binding a part 20 and a part 21 shown in Fig. 12.

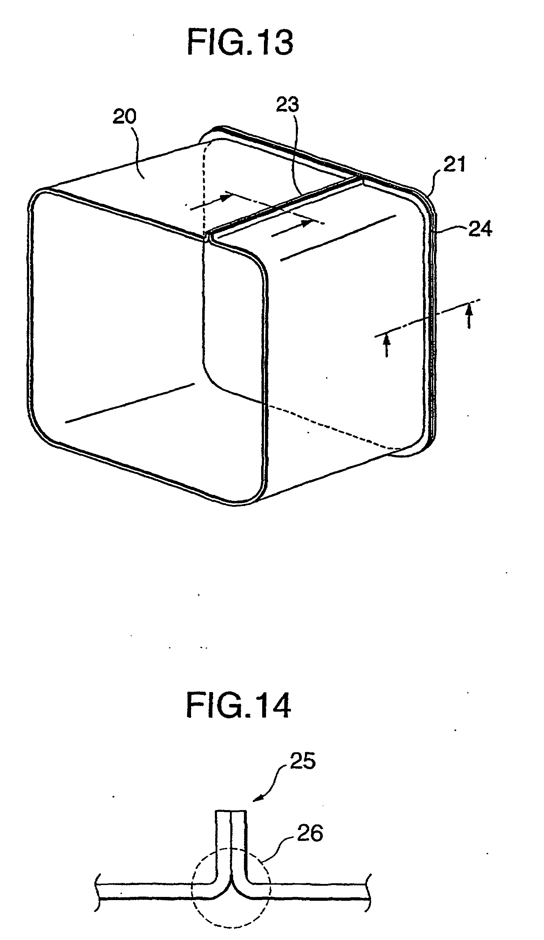

A welded joint portion 25 is configured as shown in Fig. 14 for facilitating the welding operation and automation. With the structure illustrated in Fig. 14, a line 23 and another line 24 in Fig. 13 are welded to be jointed together.

However, this method produces a narrow gap at a portion 26 as shown in Fig. 14.

When this portion 26 is weld-brazed to obtain a flat shape, the structure is strained by heat and an amount of weld heat is increased, which results in a large variation in material of the structure.

Furthermore, the water which has entered the narrow gap such as the portion 26 is oxidative, and even the prior art stainless may generate a defect due to corrosion when it is used for a long period of time.

When any material which can be a source of contamination enters this gap, contamination occurs even if the material has an antibacterial activity, and corrosion is encouraged when a corrosive material enters here.

When these extraneous substances once entered, it is hard to remove them because the gap is narrow.

That is, even if an antibacterial material is used, the contaminant may be accumulated depending on the interior structure, thus disabling demonstration of the antibacterial action.

### SUMMARY OF THE INVENTION

The present invention proposes a countermeasure for a joint portion which can be a breeding area for contamination and corrosion.

The present invention provides a interior housing of a humidistat coupled by welding, wherein a welded portion is welded by an edge joint structure.

Further, the present invention provides the interior housing of a humidistat coupled by welding, wherein the welded portion is butt-welded.

Furthermore, the present invention provides the interior housing of a humidistat coupled by welding, wherein the welded portion is provided at any position except a corner portion of the housing.

In addition, the present invention provides the interior housing of a humidistat connected by welding, wherein the corner portion of the housing is curved.

Moreover, according to the present invention, the interior housing is produced by an antibacterial steel sheet (antibacterial stainless).

The present invention provides an interior structure of an incubator, a thermostat/humidistat or a food high humidifier capable of holding a humidity environment in a chamber to 70%RH or above, wherein a shape of a coupled joint portion is obtained by butt welding or by welding using an edge joint structure in order to form the interior structure by using a stainless steel sheet having a copper content of not less than 1% and not more than 15% or a steel sheet having a copper content of not less than 1% and not more than 15%.

Further, according to the present invention, at least four corner portions of the interior housing are curved.

### BRIEF DESCRIPTION OF THE DRAWINGS

Fig. 1 is a view for explaining the state before welding according to a first embodiment;
Fig. 2 is a view for explaining the state after welding according to the first embodiment;
Fig. 3 is a view showing a cross section taken along a line A-A' in Fig. 2;
Fig. 4 is a view showing a cross section taken along a line B-B' in Fig. 2;
Fig. 5 is a view for explaining a second embodiment according to the present invention;
Fig. 6 is a perspective view for explaining a third embodiment according to the present invention;
Fig. 7 is a perspective view for explaining a fourth embodiment according to the present invention;
Fig. 8 is a perspective view for explaining a fifth embodiment according to the present invention;
Fig. 9 is a perspective view for explaining a sixth embodiment according to the present invention;
Fig. 10 is a perspective view for explaining a seventh embodiment according to the present invention;
Fig. 11 is a perspective view for explaining an incubator;
Fig. 12 is a perspective view for explaining a prior art interior housing before welding;
Fig. 13 is a view for explaining the prior art interior housing after welding; and
Fig. 14 is a view for explaining a cross section at a portion indicated by a broken line in Fig. 13.

### DETAILED DESCRIPTION OF THE PREFERRED EMBODIMENT

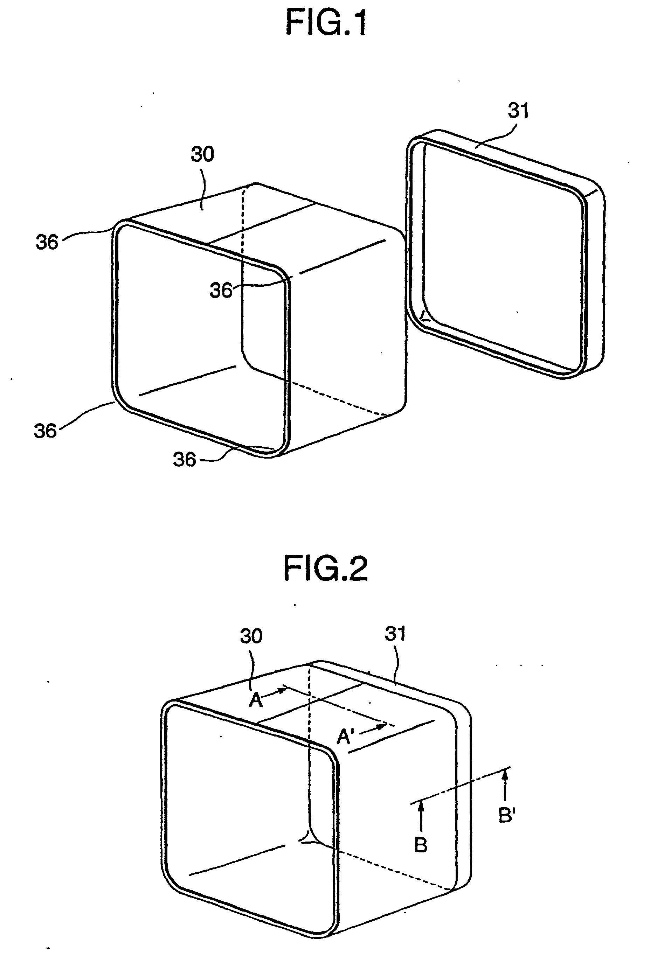

A first embodiment according to the present invention will now be described with reference to Figs. 1 to 4.

A portion 30 and another portion 31 are formed of a steel sheet containing copper.

Corner portions 36 of the portion 30 are curved by a bending process.

The portion 31 has all the corner portions curved by a drawing process using a die and the like.

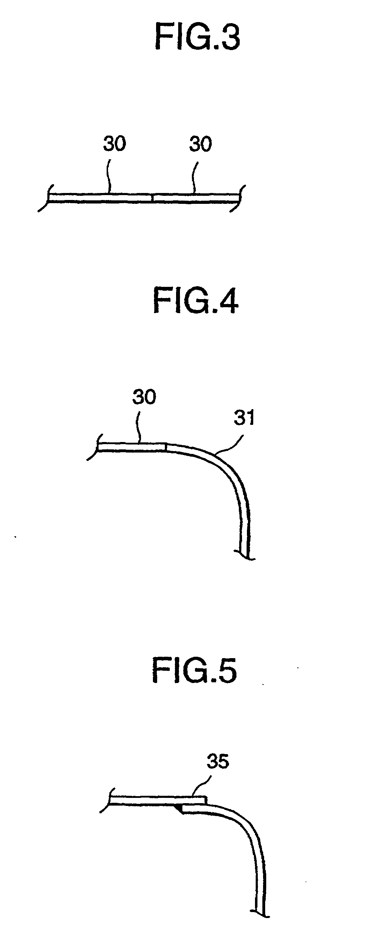

When coupling the both ends of the portion 30, they are coupled by butt welding as shown in Fig. 3 so as not to produce a deep hollow gap.

Further, when coupling the portions 30 and 31, they are coupled by butt welding as shown in Fig. 4 so as not to produce a deep hollow gap.

The above-described process can obtain the curved corner portions which can be easily cleaned and the welded portions having no gap to which contaminant gains entrance, and the antibacterial action realized by the copper component contained in the steel sheet can be effectively demonstrated/maintained, providing the corrosion-resisting structure.

Fig. 5 shows a second embodiment according to the present invention.

In the second embodiment, the edge joint welding substitutes for the butt welding shown in Fig. 4 according to the first embodiment.

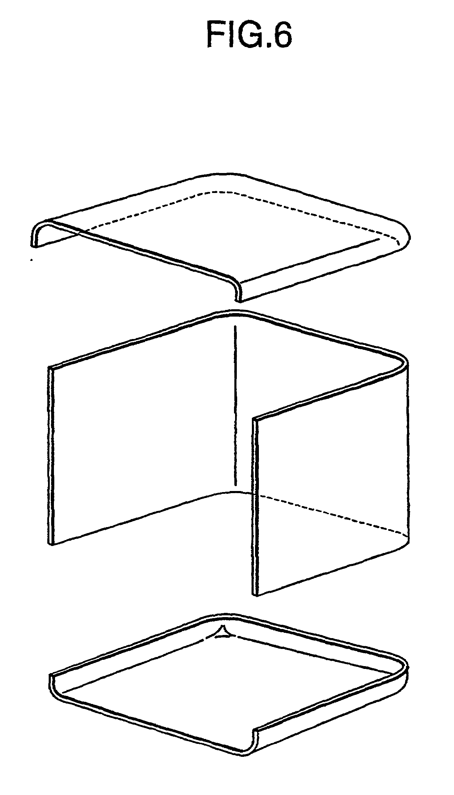

Fig. 6 shows a third embodiment.

This is an example where the welded portion is modified.

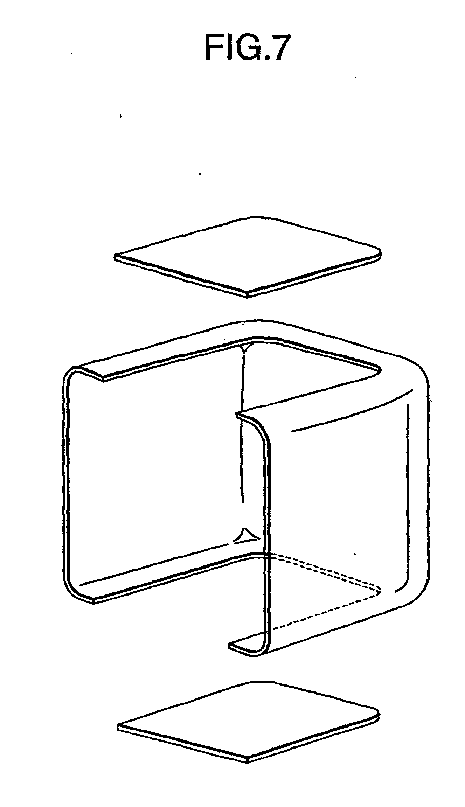

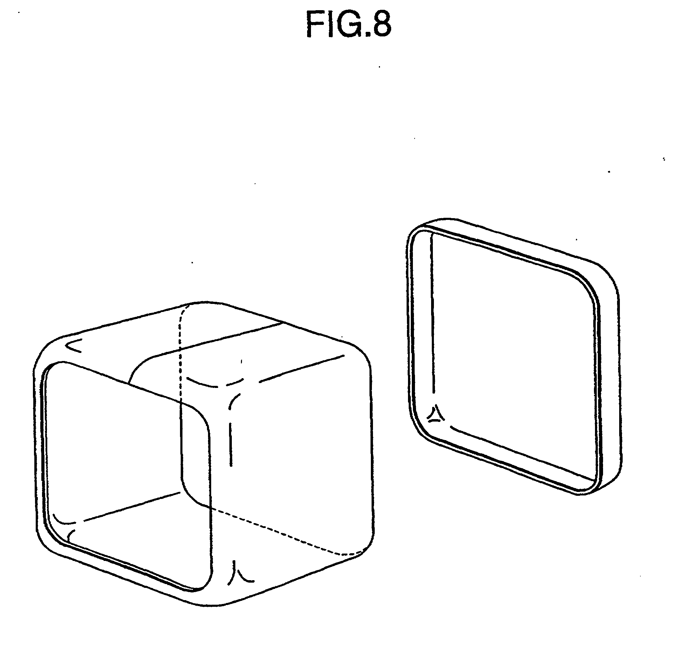

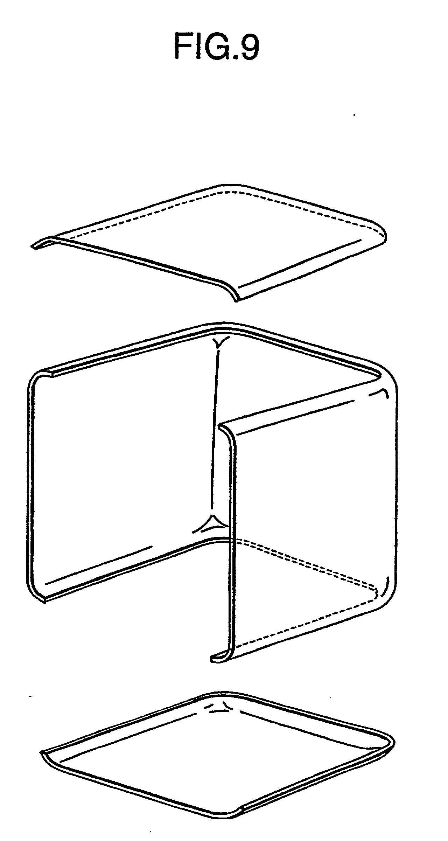

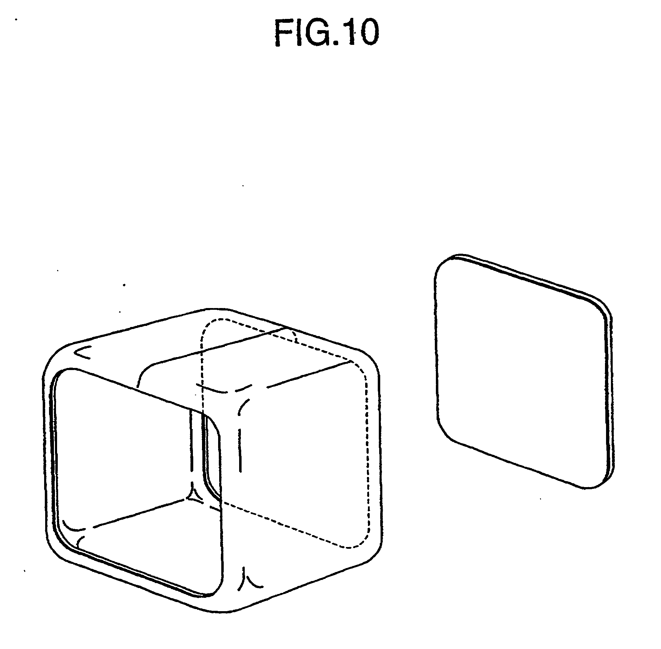

Similarly, Figs. 7 to 10 respectively show fourth to seventh embodiments in which the welded portion is varied.

According to the foregoing embodiments, when a shape of the welded joint portion is obtained by the butt welding or welding using the edge joint structure in order to realize the interior housing, the antibacterial characteristic assured by the copper component can be constantly maintained in the interior housing. In addition, there is also provided a durable incubator or an environmental tester or a food preserver adopting a corrosion-inhibiting welded structure.

Moreover, when at least four or more corner portions in the chamber are curved by bending, drawing or welding, the water or contaminant is prevented from being stored at the corners in the chamber, and cleaning at the corner portions can be facilitated. Accordingly, the antibacterial activity obtained by the copper component and the structure that user can easily keep clean can encourage the clean interior of the chamber and the corrosion-inhibiting usage with the multiplier effect.

According to the present invention, the antibacterial characteristic and the corrosion resistance of the interior housing can be maintained at the high level.

## Claims

1. An interior housing enclosing a humidity chamber for an appliance such as a humidistat, incubator, food preserver or the like, the housing being composed of steel sheet portions (30, 31) joined together by welding along weld joints, **characterized in that** each weld joint is a butt weld joint or an edge weld joint.

2. An interior housing enclosing a humidity chamber for an appliance such as a humidistat, incubator, food preserver or the like, the housing being composed of steel sheet portions (30, 31) joined together by welding along weld joints, **characterized in that** each weld joint extends with a distance from any edge portion or corner portion (36) of the housing.

3. A housing as claimed in claim 1 or 2, wherein all edge portions or corner portions (36) of the housing have a curved shape.

4. A housing as claimed in any of claims 1 to 3, wherein said steel sheet portions (30, 31) are made of antibacterial steel.

5. A housing as claimed in claim 4, wherein said antibacterial steel is antibacterial stainless steel.

## Patentansprüche

1. Innengehäuse als Umschließung einer Feuchtigkeitskammer für ein Gerät wie z.B. Feuchtklimakammer, Brotschrank, Nahrungskonservierer od. dgl., wobei das Gehäuse aus Stahlblechteilen (30, 31) zusammengesetzt ist, die durch Schweißen längs Schweißnähten miteinander verbunden sind, **dadurch gekennzeichnet, daß** jede Schweißnaht eine Stumpfschweißnaht oder eine Überlappungsschweißnaht ist.

2. Innengehäuse als Umschließung einer Feuchtigkeitskammer nach Anspruch 1, **dadurch gekennzeichnet, daß** jede Schweißnaht in einem Abstand von jedem Kantenbereich oder Eckbereich (36) des Gehäuses verläuft.

3. Gehäuse nach Anspruch 1 oder 2,
bei dem alle Kantenbereiche oder Eckbereiche (36) des Gehäuses eine gekrümmte Form haben.

4. Gehäuse nach einem der Ansprüche 1 bis 3,
bei dem die Stahlblechteile (30, 31) aus antibakteriellem Stahl sind.

5. Gehäuse nach Anspruch 4,
bei dem der antibakterielle Stahl ein antibakterieller Edelstahl ist.

## Revendications

1. Logement intérieur enfermant une chambre d'humidité pour un appareil domestique tel qu'un régulateur d'humidité, un incubateur, un conservateur d'aliment ou analogues, le logement étant constitué de parties de feuille d'acier (30, 31) raccordées ensemble par soudure le long de joints de soudure, **caractérisé en ce que** chaque joint de soudure est un joint de soudure bout à bout ou un joint de soudure de bord.

2. Logement intérieur enfermant une chambre d'humidité pour un appareil domestique tel qu'un régulateur d'humidité, un incubateur, un conservateur d'aliment ou analogues, le logement étant constitué de parties de feuille d'acier (30, 31) raccordées ensemble par soudure le long de joints de soudure, **caractérisé en ce que** chaque joint de soudure s'étend à une certaine distance à partir de chaque partie de bord ou partie de coin (36) du logement.

3. Logement selon la revendication 1 ou 2, dans lequel la totalité des parties de bord ou des parties de coin (36) du logement ont une forme incurvée.

4. Logement selon l'une quelconque des revendications 1 à 3, dans lequel lesdites parties de feuille d'acier (30, 31) sont constituées d'acier antibactérien.

5. Logement selon la revendication 4, dans lequel ledit acier antibactérien est un acier inoxydable antibactérien.
